# FASCICULE DE BREVET EUROPEEN

(11) **EP 1 850 863 B1**
(45) Date de publication et mention de la délivrance du brevet: **16.02.2011**
(21) Numéro de dépôt: 05850558.7
(22) Date de dépôt: 21.12.2005
(51) Int. Cl.: A61K 38/48, A61P 23/02

(54) **UTILISATION DE TOXINE BOTULIQUE POUR UNE INSENSIBILISATION LOCALE PROLONGÉE**
VERWENDUNG VON BOTULINUMTOXIN FÜR DIE VERLÄNGERTE LOKALE DESENSIBILISIERUNG
USE OF BOTULINUM TOXIN FOR PROLONGED LOCAL DESENSITIZATION

(30) Priorité: 21.12.2004 FR 0413650
(43) Date de publication de la demande: 07.11.2007
(73) Titulaire: IPSEN PHARMA, 92100 Boulogne-Billancourt (FR)
(72) Inventeur: CHERIF-CHEIKH, Roland, E-08860 Castelldefels (ES)
(74) Mandataire: Retzler, Charlotte
(86) Numéro de dépôt international: PCT/FR2005/003209
(87) Numéro de publication internationale: WO 2006/067332

(56) Documents cités:
- WO-A-01/78760
- WO-A-2004/006954
- US-A- 5 589 192
- CUI MINGLEI ET AL: "Subcutaneous administration of botulinum toxin A reduces formalin-induced pain." PAIN, vol. 107, no. 1-2, janvier 2004 (2004-01), pages 125-133, XP002337053 ISSN: 0304-3959

## Description

La présente invention concerne l'utilisation de toxine botulique pour une insensibilisation locale prolongée.

La toxine botulique, en particulier la toxine botulique de type A (Dysport^{®} commercialisé par Ipsen ou Botox^{®} commercialisé par Allergan), est utilisée depuis les années 80 chez l'homme pour le traitement de maladies / désordres divers et variés. Parmi les maladies / désordres pouvant être traités par la toxine botulique, on peut citer entre autres des désordres musculaires (par exemple le blépharospasme, la spasticité de l'adulte ou de l'enfant ou encore le torticolis), la migraine, la douleur d'origine musculaire, la douleur neuropathique, le diabète, l'hyperhidrose (ou transpiration excessive), l'hypersalivation ou même les rides.

Les applications des toxines botuliques connues à ce jour ont trait à son administration classique, intra-musculaire telle que décrit dans les traitements mentionnés. La réduction de la douleur pour les applications concernées, est obtenue comme pour l'ensemble des applications thérapeutiques de ces toxines, après injection dans les muscles par leur paralysie transitoire, c'est à dire en bloquant les contractions musculaires sur une certaine période de temps. C'est donc généralement indirectement à travers le contrôle des spasmes ou contractions musculaires que l'on contrôle la douleur.

Il existe de nombreuses pathologies avec des localisations de la douleur au niveau de la peau et / ou du tissu sous-cutané qui pourraient avantageusement bénéficier d'un traitement local prolongé et renouvelable. C'est le cas par exemple des douleurs provoquées par des injections de formes retard, comme par exemple les injections d'autogel, les dépôts et implantations sous-cutanés (pompes, pacemaker, etc.). De même, certains traitements thérapeutiques nécessitent l'injection régulière de substances médicamenteuses, sous forme par exemple de piqûres quotidiennes. C'est le cas notamment des traitements du diabète insulino-dépendant, où le patient s'injecte au minimum une fois par jour, mais plus souvent après chaque repas, une dose d'insuline. C'est le cas par exemple de certains traitements qui nécessitent des injections quotidiennes d'hormone de croissance. C'est aussi le cas par exemple de certains traitements du cancer, qui nécessitent des injections répétées d'un agent anti-cancéreux. C'est également le cas des prélèvements sanguins répétés, comme ceux visant au dosage du glucose sanguin. Or cela est souvent fort douloureux pour les patients dont certains finissent par développer une aversion pour les piqûres ou toutes les formes d'injections. L'ensemble de ces douleurs, mentionnées ci-dessus, est appelé selon la présente invention douleurs induites, répétitives ou ponctuelles répétées. Il s'agit d'une douleur provoquée par une intervention extérieure, par un stimuli ou par un traumatisme invasif au niveau du derme ou de la peau ou du tissu sous-cutané. La douleur peut par exemple être due à l'injection elle-même, notamment l'incision du derme par l'aiguille, ou elle peut également être due au produit injecté lui-même.

Dans ces cas, comme pour toutes les autres douleurs induites, répétitives, ponctuelles ou prolongées au niveau de la peau et du tissu sous-cutané, il apparaît souhaitable de disposer d'un traitement anesthésique prolongé et localisé. A la connaissance de la Demanderesse, aucune solution efficace et durable n'a toutefois été trouvée à ce jour pour remédier à ces problèmes.

La Demanderesse vient justement de découvrir que la toxine botulique peut être utilisée pour préparer un médicament destiné à insensibiliser de façon réversible une zone de peau, ladite zone de peau étant sujette à des douleurs induites, répétitives ou ponctuelles répétées occasionnées par des piqûres ou des injection répétées. Ainsi lesdites douleurs induites, répétitives ou ponctuelles répétées sont évitées ou traitées. Ces douleurs peuvent notamment correspondre à un traitement, à des piqûres répétées (liées par exemple à des administrations répétées d'insuline, d'hormone de croissance, d'agents de chimiothérapie ou d'autres médicaments sous forme injectable ou encore à des piqûres de prélèvement de sang, par exemple en vue de la mesure de taux de glucose sanguins) ou bien à la mise en place d'implants, de dispositifs implantables (par exemple pompes, pacemakers, etc.) ou de systèmes d'administration de substances thérapeutiques (par exemple les perfusions).

La présente invention a donc pour objet l'utilisation de toxine botulique ou d'un fragment de toxine botulique possédant l'activité de ladite toxine pour préparer un médicament destiné à insensibiliser de façon réversible une zone de peau, ladite zone de peau étant sujette à des douleurs induites, répétitives ou ponctuelles répétées occasionnées par des piqûres ou des injection répétées.

Selon cette nouvelle utilisation selon l'invention, la toxine botulique sera de préférence injectée directement par la voie intra-dermique, trans-dermique ou sous-cutanée dans la zone à traiter et non pas par la voie intra-musculaire comme d'ordinaire. Le cas échéant, cette administration pourra prendre la forme de l'application d'un patch ou d'une composition pour application topique (par exemple, crème, gel, lotion, etc.) comprenant de la toxique botulique.

La toxine botulique utilisée pour la préparation d'un médicament selon l'invention sera de préférence choisie parmi les toxines botuliques de type A (incluant A₁, A₂ et A₃), B, C (incluant Ci et C₂), D, E, F ou G. De préférence, elle sera choisie parmi les toxines botuliques de type A, B ou F. Encore plus préférentiellement, elle sera choisie parmi les toxines botuliques de type A ou B ; en particulier, il s'agira de la toxine botulique de type A. Alternativement, il pourra s'agir d'un fragment de toxine botulique possédant l'activité de ladite toxine, ou encore d'un analogue de la toxine botulique possédant l'activité de ladite toxine.

Par ailleurs, lorsqu'une toxine botulique de type A, B, C, D, E, F ou G sera utilisée pour la préparation d'un médicament selon l'invention, ladite toxine pourra se présenter sous forme d'un complexe comprenant la toxine botulique ou alors sous forme libre (i.e. libre de toute protéine la complexant).

Selon l'invention, le médicament préparé pourra être une poudre lyophilisée comprenant la toxine botulique (auquel cas le médecin reconstituera la solution avec de l'eau ou une solution aqueuse saline avant de l'injecter au patient) ou encore une solution injectable comprenant ladite toxine (telle par exemple celle décrite dans la demande de brevet WO 00/15245).

Selon une variante préférentielle, la présente invention concerne l'utilisation de toxines botuliques ou d'un fragment de toxine botulique possédant l'activité de ladite toxine pour préparer un médicament destiné à insensibiliser de façon réversible une zone de peau, ladite zone de peau étant sujette à des douleurs induites, répétitives ou ponctuelles répétées provoquées par l'administration d'insuline, d'hormone de croissance, de peptides sous forme "autogel", d'agents de chimiothérapie ou d'autres médicaments sous forme injectable ou encore sont provoquées par des piqûres de prélèvement de sang en vue de la mesure de taux de glucose sanguin.

Par peptides sous forme "autogel", on entend au sens de la présente invention une formulation retard de peptides, comme par exemple la formulation d'analogue de la somatostatine (Somatuline forme "autogel" 60, 90 ou 120 mg).

Par insuline, on entend toute insuline, qu'elle soit à action très rapide, rapide, intermédiaire, lente ou très lente, ou encore un mélange de telles insulines. Par insuline à action très rapide, on entend notamment l'insuline lispro (i.e. l'analogue de l'insuline constituant le principe actif de Humalog^{®}). Par insuline à action rapide, on entend notamment des insulines humaines recombinantes telles que le principe actif de Humulin^{®} R (Eli Lilly) ou de Novolin^{®} ge Toronto (Novo Nordisk). Par insuline intermédiaire, on entend en particulier l'insuline Neutral Protamine Hagedorn ou insuline NPH (et par exemple le principe actif de Humulin^{®} N ou Humulin^{®} L (Eli Lilly) ou de Novolin^{®} ge NPH ou Novolin^{®} ge Lente (Novo Nordisk)). Par insuline à action lente ou très lente, on entend notamment l'insuline glargine (i.e. l'analogue de l'insuline constituant le principe actif de Lantus^{®}), les insulines associées à des ions Zn²⁺ ou le principe actif de Humulin^{®} U (Eli Lilly) ou de Novolin^{®} ge Ultralente (Novo Nordisk).

Par hormone de croissance, on entend toute hormone de croissance naturelle ou synthétique (et par exemple une hormone de croissance recombinante), et notamment l'hormone de croissance commercialisée en Europe sous la marque NutropinAq^{®}.

Par piqûres ou injection répétées, on entend des piqûres ou des injections quotidiennes ou hebdomadaires ou bi-mensuelles, de préférence qui ont lieu dans un périmètre de peau du patient compris entre 1 et 5 cm². De préférence les piqûres ou injection répétées sont des piqûres sous-cutanées ou injections sous-cutanées.

Par l'expression "insensibiliser de façon réversible une zone de peau", on entend au sens de la présente invention une anesthésie locale de la zone de peau. Aussi la présente invention ne vise pas à traiter la douleur mais à anesthésier une zone de peau.

Selon l'invention, la zone de peau insensibilisée pourra être créée par l'injection sous-cutanée de toxine botulique en un seul point, ou de préférence, par l'injection sous-cutanée de toxine botulique en plusieurs points définissant une figure géométrique (par exemple un triangle équilatéral, un carré, un pentagone régulier, un hexagone régulier, etc.) ou équitablement répartis sur une zone de peau (par exemple réseau de points répartis).

De préférence, la zone traitée sera la plus réduite possible et limitée aux besoins de contrôle de la douleur. Pour le cas d'injections répétées par exemple, il pourra s'agir d'une zone de 1 à 5 centimètres carrés de surface de peau associée, de préférence de 1 à 3 cm² ou non à une zone de 1 à quelques centimètres de profondeur de tissu sous-cutané.

Pour le cas particulier de personnes qui auraient à procéder à des piqûres de prélèvement de sang en vue de la mesure de taux de glucose sanguins, l'on pourra par exemple envisager de procéder à une injection ou des injections de toxine botulique dans un ou plusieurs doigts afin d'insensibiliser des zones destinées à recevoir des piqûres correspondantes d'aiguilles ou de lancets.

De préférence, l'insensibilisation locale créée par l'injection de toxine botulique durera au moins un mois, et plus préférentiellement au moins 2, 3 ou même 6 mois.

La variation de la dose et de la dilution permettra de jouer sur l'activité et la diffusion de la toxine botulique et par conséquent sur la partie de la peau et des tissus sous-cutanés insensibilisée ainsi que sur l'intensité du traitement.

De préférence les doses injectées seront comprises entre 10 et 200 Unités de toxine botulique, plus préférentiellement de 20 à 150 Unités de toxine botulique, encore plus préférentiellement de 50 à 100 Unités de toxine botulique.

Afin d'éviter des lésions aux tissus au voisinage de la zone traitée par la toxine botulique et subissant les piqûres régulières, l'on changera de préférence régulièrement de zone pour le traitement (en tout état de cause au plus tard au moment où l'insensibilisation produite par la toxine botulique cesse de produire ses effets) ; par exemple, dans le cas de la toxine botulique de type A, l'on peut prévoir de changer de zone environ 3 mois après administration de la toxine. Dans cette situation, on administrera donc la dose appropriée de toxine botulique à la nouvelle zone devant être insensibilisée de 1 à 7 jours avant de recevoir les piqûres régulières, ceci afin de laisser à la toxine botulique le temps de produire ses effets.

Le traitement sera répété en fonction des besoins du patient. Il se peut toutefois que cette aide à la tolérance locale lors du début du traitement ait été suffisante et que le patient ne nécessite plus ensuite ce traitement ou qu'il soit possible d'en diminuer progressivement la dose. De préférence donc ces injections locales de toxine botulique seront pratiquées peu de temps avant (par exemple de 1 à 10 jours) ou au commencement (c'est-à-dire le jour même du commencement) de traitements chroniques par injection ou encore peu de temps avant (par exemple de 1 à 10 jours) ou au moment (c'est-à-dire le jour même) de réaliser une implantation sous-cutanée douloureuse de dispositif tel que pompe, pacemaker, prothèse mammaire ou autre.

Dans le cas de douleurs pathologiques autres à ce niveau, le traitement pourra aussi être une réponse curative et non plus un traitement préventif.

La présente utilisation selon l'invention exclut le traitement des douleurs provoquées par la formaline ou l'arthrite ou une tumeur quelconque ou une brûlure ou une migraine. L'utilisation selon l'invention exclut également le traitement des douleurs inflammatoires ou post-opératoires ou viscérales.

Selon une variante de l'invention, le patient dont une zone de peau sera insensibilisée de façon réversible pourra être un animal. De préférence, ledit patient sera un être humain (homme ou femme).

A moins qu'ils ne soient définis d'une autre manière, tous les termes techniques et scientifiques utilisés ici ont la même signification que celle couramment comprise par un spécialiste ordinaire du domaine auquel appartient cette invention.

Les exemples suivants sont présentés pour illustrer les procédures ci-dessus et ne doivent en aucun cas être considérés comme une limite à la portée de l'invention.

### EXEMPLES

### Exemple 1

Un patient diabétique doit régulièrement s'injecter par la voie sous-cutanée de l'insuline. Le patient reçoit par la voie sous-cutanée 40 unités Ipsen de toxine botulique (Dysport^{®}; fournisseur : Ipsen Limited, Royaume-Uni ; préparation selon les recommandations du fabricant) réparties équitablement en 4 points définissant un carré de 2 cm repéré sur la peau du patient. Après quelques jours, les injections sous-cutanées d'insuline effectuées dans le carré prétraité par la toxine botulique deviennent bien moins douloureuses pour le patient. Trois mois plus tard, la procédure est répétée en choisissant un autre carré de peau comme zone à insensibiliser.

### Exemple 2

Un enfant de 8 ans se voit prescrire des injections sous-cutanées journalières d'hormone de croissance. Une semaine avant de débuter le traitement avec l'hormone de croissance, 30 unités Ipsen de toxine botulique (Dysport^{®} ; fournisseur : Ipsen Limited, Royaume-Uni ; préparation selon les recommandations du fabricant) sont réparties équitablement et injectées par la voie sous-cutanée en 4 points définissant un carré de 2 cm repéré sur la peau du patient. Une semaine plus tard, les injections sous-cutanées journalières d'hormone de croissance sont effectuées dans les limites du carré prétraité par la toxine botulique sans que l'enfant ne ressente de véritable douleur liée aux injections. Trois mois plus tard, la procédure est répétée en choisissant un autre carré de peau comme zone à insensibiliser.

### Exemple 3

Un patient diabétique est traité avec 10 unités Ipsen de toxine botulinique Dysport^{®} ou sur un même micro volume, avec du sérum physiologique placebo en différents sites de piqûre pour prélever une goutte de sang avec un dispositif type lancet permettant ensuite de mesurer son taux de glucose sanguin par exemple aux extrémités des doigts.

Les sites d'injection de Dysport^{®} et de placebo sont ensuite exposés et évalués par le patient selon une échelle de douleur de 1 à 6.

Il ressort de cette étude une différence significative avec diminution de la douleur perçue aux sites traités par le Dysport^{®} (toxine botulique) sur une période de 3 mois et plus.

### Exemple 4

Un patient soufrant d'acromégalie est traité avec une forme retard Autogel en sous cutanée à raison d'une injection de 120 mg de lanréotide tous les mois (Somatuline 120 mg de chez Ipsen). Il évalue selon une échelle, la douleur de l'injection. Les sites habituels d'injection sont traités par une injection de 40 unités Ipsen de toxine botulinique Dysport^{®} et la douleur du traitement est évaluée par différence avec la référence.

Il ressort de cette étude que les 6 injections suivantes dans la zone traitée, soit sur 6 mois de traitement, sont significativement perçues comme moins douloureuses qu'avant admistration de Dysport^{®}.

## Revendications

1. Utilisation de toxine botulique ou d'un fragment de toxine botulique possédant l'activité de ladite toxine pour préparer un médicament destiné à insensibiliser de façon réversible une zone de peau, ladite zone de peau étant sujette à des douleurs induites, répétitives ou ponctuelles répétées occasionnées par des piqûres ou des injections répétées.

2. Utilisation selon la revendication 1 **caractérisé en ce que** les douleurs sont provoquées par l'administration d'insuline, d'hormone de croissance, de peptides sous forme "autogel", d'agents de chimiothérapie ou d'autres médicaments sous forme injectable ou encore sont provoquées par des piqûres de prélèvement de sang en vue de la mesure de taux de glucose sanguin.

3. Utilisation selon la revendication 1 ou 2, **caractérisée en ce que** l'on utilise une toxine botulique choisie parmi les toxines botuliques de type A, B, C, D, E, F ou G.

4. Utilisation selon la revendication 3, **caractérisée en ce que** la toxine botulique utilisée est choisie parmi les toxines botuliques de type A, B ou F.

5. Utilisation selon la revendication 4, **caractérisée en ce que** la toxine botulique utilisée est choisie parmi les toxines botuliques de type A ou B.

6. Utilisation selon la revendication 5, **caractérisée en ce que** la toxine botulique utilisée est la toxine botulique de type A.

7. Utilisation selon l'une des revendications 1 à 6, **caractérisée en ce que** médicament préparé est destiné à être administré par la voie intra-dermique, trans-dermique ou sous-cutanée.

8. Utilisation selon l'une des revendications précédentes, **caractérisée en ce que** l'insensibilisation provoquée dure au moins un mois.

9. Utilisation selon la revendication 8, **caractérisée en ce que** l'insensibilisation provoquée dure au moins deux mois.

10. Utilisation selon la revendication 9, **caractérisée en ce que** l'insensibilisation provoquée dure au moins trois mois.

## Claims

1. Use of botulinum toxin or a fragment of botulinum toxin possessing the activity of said toxin for preparing a medicament intended to desensitize an area of skin in a reversible fashion, said area of skin being subject to repeated, repetitive or specific induced pain caused by repeated punctures or injections.

2. Use according to claim 1 **characterized in that** the pain is caused by the administration of insulin, growth hormone, peptides in the form of "autogel", chemotherapy agents or other medicaments in injectable form or also caused by punctures for blood collections for measuring blood glucose level.

3. Use according to claim 1 or 2, **characterized in that** a botulinum toxin chosen from the botulinum toxins of type A, B, C, D, E, F or G is used.

4. Use according to claim 3, **characterized in that** the botulinum toxin used is chosen from the botulinum toxins of type A, B or F.

5. Use according to claim 4, **characterized in that** the botulinum toxin used is chosen from the botulinum toxins of type A or B.

6. Use according to claim 5, **characterized in that** the botulinum toxin used is chosen from the botulinum toxin of type A.

7. Use according to one of the claims 1 to 6, **characterized in that** the medicament prepared is intended to be administered by intradermal, transdermal or subcutaneous route.

8. Use according to one of the preceding claims, **characterized in that** the desensitization caused lasts at least one month.

9. Use according to claim 8, **characterized in that** the desensitization caused lasts at least two months.

10. Use according to claim 9, **characterized in that** the desensitization caused lasts at least three months.

## Patentansprüche

1. Verwendung von Botulinumtoxin oder eines Botulinumtoxinfragments, welches Botulinumtoxinaktivität aufweist, zur Herstellung eines Medikaments, das zur reversiblen Desensibilisierung einer Hautzone bestimmt ist, wobei die Hautzone wiederholten induzierten, repetitiven oder punktuellen, durch wiederholtes Spritzen oder wiederholte Injektionen hervorgerufenen Schmerzen ausgesetzt ist.

2. Verwendung nach Anspruch 1, **dadurch gekennzeichnet, dass** die Schmerzen verursacht werden durch die Verabreichung von Insulin, Wachstumshormon, Peptiden, die als « Autogel » vorliegen, chemotherapeutischen Agenzien, oder anderen injizierbaren Medikamenten, oder auch durch Blutabnahmespritzen zum Messen des Blutglukosegehalts.

3. Verwendung nach Anspruch 1 oder 2, **dadurch gekennzeichnet, dass** ein Botulinumtoxin verwendet wird, das ausgewählt ist unter Botulinumtoxinen des Typs A, B, C, D, E, F oder G.

4. Verwendung nach Anspruch 3, **dadurch gekennzeichnet, dass** das verwendete Botulinumtoxin ausgewählt ist unter Botulinumtoxinen des Typs A, B oder F.

5. Verwendung nach Anspruch 4, **dadurch gekennzeichnet, dass** das verwendete Botulinumtoxin ausgewählt ist unter Botulinumtoxinen des Typs A, B oder F.

6. Verwendung nach Anspruch 5, **dadurch gekennzeichnet, dass** das verwendete Botulinumtoxin Botulinumtoxin Typs A ist.

7. Verwendung nach einem der Ansprüche 1 bis 6, **dadurch gekennzeichnet, dass** das Medikament zur intradermalen, transdermalen oder subkutanen Verabreichung vorgesehen ist.

8. Verwendung nach einem der vorhergehenden Ansprüche, **dadurch gekennzeichnet, dass** die ausgelöste Desensibilisierung wenigstens einen Monat dauert.

9. Verwendung nach Anspruch 8, **dadurch gekennzeichnet, dass** die verursachte Desensibilisierung wenigstens zwei Monate dauert.

10. Verwendung nach Anspruch 9, **dadurch gekennzeichnet, dass** die verursachte Desensibilisierung wenigstens drei Monate dauert.
